(19) **Europäisches Patentamt**
**European Patent Office**
**Office européen des brevets**

(11) **EP 2 826 420 B1**

(12) **EUROPEAN PATENT SPECIFICATION**

(45) Date of publication and mention of the grant of the patent:
**27.02.2019 Bulletin 2019/09**

(51) Int Cl.:
*A61B 5/08* *(2006.01)*       *A61B 5/00* *(2006.01)*
*A61B 5/145* *(2006.01)*

(21) Application number: **14177096.6**

(22) Date of filing: **15.07.2014**

(54) **Apparatus for predicting change in physical index**

Vorrichtung zur Vorhersage der Änderung eines körperlichen Index

Appareil pour prédire les changements dans un index physique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.07.2013 JP 2013147738**

(43) Date of publication of application:
**21.01.2015 Bulletin 2015/04**

(73) Proprietor: **Tanita Corporation**
**Tokyo 174-8630 (JP)**

(72) Inventors:
• **Kodama, Miyuki**
  **Itabashi-ku, Tokyo 174-8630 (JP)**

• **Sano, Ayumi**
  **Itabashi-ku, Tokyo 174-8630 (JP)**

(74) Representative: **Epping - Hermann - Fischer Patentanwaltsgesellschaft mbH**
**Schloßschmidstraße 5**
**80639 München (DE)**

(56) References cited:
WO-A1-03/079890       WO-A1-2007/062663
WO-A1-2013/046810     WO-A2-2004/088304
JP-A- 2001 349 888    US-A1- 2007 083 335
US-B1- 6 506 152

**Description**

TECHNICAL FIELD

The present invention relates to an apparatus for predicting change in a subject's physical index based on the ketone body concentration excreted by the subject.

BACKGROUND ART

When going on a healthy diet, people hope to reduce their body fat. Therefore, there is a desire to ascertain the amount of body fat itself as well as the reduction in body fat due to various activities such as a particular exercise, dietary restrictions, or regular daily activities. JP2001-349888A thus proposes measuring the amount of burned body fat based on breath acetone concentration. Based on the measured amount of burned body fat, it is possible to manage the reduction in body fat. The international patent application WO 03/079890 A1 discloses a weight loss system and a method for measuring one or more metabolic parameters in a body fluid sample. Moreover WO 2007/062663 A1 relates to a method to obtain a regulation of an energy balance wherein the method provides a user with a suggested energy input value. WO 2013/046810 A1 provides a weight management device. The patent document US 6,506,152 B1 teaches a caloric energy balance monitor using calories expended during rest and activity. A health-monitoring device assessing the health of a user based on levels of two analytes in a biological fluid is disclosed by US 2007/083335 A1. A first analyte indicative of fat metabolism and a second analyte of indicative of glucose metabolism are utilized. The levels of the two analytes may be used to assess insulin sensitivity. Moreover also the international application WO 2004/088304 A2 proposes a health-monitoring device assessing the health of a user based on levels of two analytes in a biological fluid.

SUMMARY OF INVENTION

People who are working hard to diet are highly interested in learning how their body weight will change as a result of exercise they are currently performing. The apparatus disclosed in JP2001-349888A reveals the reduction in body fat at the time of measurement, yet change is not apparent over a short period of time. Rather, measurement over a certain extended period of time is necessary, and hence this apparatus cannot predict the future change in fat, body weight, or the like.

The present invention has been conceived in light of the above circumstances, and it is an object thereof to provide an apparatus for predicting a future change in a physical index by recognizing indications of change relating to a subject's physical index over a short period of time.

The invention is set out in the appended claims. In order to resolve the above-described problems, an apparatus for predicting change in a physical index according the present invention comprises: an acquisition unit configured to acquire a ketone body concentration excreted by a subject; a setting unit configured to set a set time at a present time or a later time; and a calculation unit configured to calculate, based on the ketone body concentration and on the set time, a predicted value of change in a physical index of the subject when the set time elapses.

The inventors discovered that a change in a physical index after any length of time correlates with the ketone body concentration excreted by a subject. Therefore, based on this correlation, the apparatus for predicting change in a physical index according to the invention can use the ketone body concentration excreted by the subject and any detected length of time to calculate the predicted value of the change in the physical index when the length of time from the present has elapsed.

An apparatus for predicting change in a physical index according to the present invention further comprises a storage unit configured to store at least one ketone body concentration acquired by the acquisition unit, and the calculation unit calculates the predicted value of change in the physical index based on a past ketone body concentration stored in the storage unit.

By calculating the predicted value based on the past ketone body concentration, the apparatus for predicting change in a physical index according to the invention can use the past ketone body concentration to reflect the tendency of change in the ketone body concentration onto the predicted value of change in the physical index, thereby improving the calculation accuracy of the predicted value.

In an apparatus for predicting change in a physical index according to the present invention, the calculation unit preferably adjusts the predicted value of change in the physical index in accordance with a change between a past ketone body concentration stored in the storage unit and a current ketone body concentration newly acquired by the acquisition unit.

By calculating the predicted value in accordance with change in the current ketone body concentration in particular with respect to one past ketone body concentration, the apparatus for predicting change in a physical index according to the invention can reflect the tendency of change in the ketone body concentration from the past to the present simply onto the predicted value of change in the physical index, thereby improving the calculation accuracy of the predicted value.

In an apparatus for predicting change in a physical index according to an embodiment, the storage unit preferably stores the ketone body concentration acquired by the acquisition unit in combination with an acquisition time, and by taking a weighted average of i) the predicted value of change in the physical index calculated based on a plurality of past ketone body concentrations and acquisition times stored in the storage unit and ii) the predicted value of change in the physical index calculated based on a current ketone body concentration newly acquired by the acquisition unit and on the set time, the calculation unit preferably calculates the predicted value of change in the physical index based on the current ketone body concentration, on the past ketone body concentrations, and on the set time.

In particular by using a plurality of past ketone body concentrations, the apparatus for predicting change in a physical index according to this embodiment predicts the current ketone body concentration and can thus reflect the tendency of change in the ketone body concentration onto the calculation of the predicted value of change in the physical index, thereby improving the calculation accuracy of the predicted value.

In an apparatus for predicting change in a physical index according to the present invention, the calculation unit determines a degree of increase in burning rate of body fat by the subject based on a change between a past ketone body concentration acquired by the acquisition unit and a current ketone body concentration newly acquired by the acquisition unit.

The apparatus for predicting change in a physical index according to the invention determines the degree of increase in burning rate of body fat by the subject, thereby allowing the subject to easily recognize the degree of current body fat burning.

In an apparatus for predicting change in a physical index according to another embodiment, the calculation unit preferably changes, in accordance with age of the subject, a threshold of change in the ketone body concentration for determining the degree of increase in burning rate.

In the apparatus for predicting change in a physical index according to this embodiment, the threshold changes due to the subject's age, which is a factor that causes the ease with which body fat is burned to vary. Therefore, the degree of increase in burning rate of body fat appropriate for the subject's age can be determined.

In an apparatus for predicting change in a physical index according to another embodiment, the calculation unit preferably changes, in accordance with the past ketone body concentration, a threshold of change in the ketone body concentration for determining the degree of increase in burning rate.

In the apparatus for predicting change in a physical index according to this embodiment, the threshold changes due to the past ketone body concentration, which is a factor that causes the ease with which body fat is burned to vary. Therefore, the degree of increase in burning rate of body fat appropriate for the subject's past ketone body concentration can be determined.

In an apparatus for predicting change in a physical index according to another embodiment, the calculation unit preferably calculates the predicted value of change in the physical index based on a product of the ketone body concentration and a length of time from the present time to the set time.

The inventors discovered that a first order or high-order polynomial having the product of ketone body concentration and length of time as a variable has a particularly high correlation with change in a physical index. Therefore, the apparatus for predicting change in a physical index according to this embodiment can calculate the predicted value of change in a physical index to a high degree of accuracy.

In an apparatus for predicting change in a physical index according to the present invention, the setting unit is an input unit configured to detect input designating the later time.

The apparatus for predicting change in a physical index according to the invention can detect the user's desired time for calculation of change in the physical index via the input unit.

According to the present invention, a predicted value of future change in the subject's body weight can be calculated.

BRIEF DESCRIPTION OF DRAWINGS

The present invention will be further described below with reference to the accompanying drawings, wherein:

FIG. 1 is an external view of an apparatus for predicting change in a physical index according to an embodiment of the present invention;
FIG. 2 is a functional block diagram schematically illustrating the internal structure of the apparatus for predicting change in a physical index in FIG. 1;
FIG. 3 is a first example of an image indicating the current state of change in body weight;
FIG. 4 is a second example of an image indicating the current state of change in body weight;
FIG. 5 is an image indicating the degree of increase in fat burning rate;

FIG. 6 illustrates a first example of an image indicating prediction of change in body weight;

FIG. 7 illustrates a second example of an image indicating prediction of change in body weight;

FIG. 8 illustrates a third example of an image indicating prediction of change in body weight;

FIG. 9 is a flowchart illustrating processing executed by the calculation unit to observe change in body weight;

FIG. 10 is a flowchart illustrating a subroutine executed by the calculation unit to determine the degree of increase in fat burning rate; and

FIG. 11 is a flowchart illustrating a subroutine executed by the calculation unit to predict change in body weight.

DESCRIPTION OF EMBODIMENTS

The following describes an embodiment of the present invention with reference to the drawings.

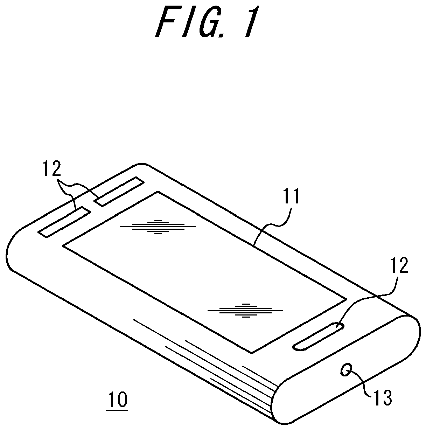

FIG. 1 is an external perspective view of an apparatus for predicting change in a physical index according to an embodiment of the present invention. In this context, a physical index refers to an index that is affected by the burning of body fat, such as an index related to body fat (for example, amount of body fat or body fat percentage), or an index related to body weight (for example, body weight).

The apparatus 10 for predicting change in a physical index may be a dedicated device that predicts change in a physical index or may be incorporated in any of a variety of devices that have other functions, such as an activity monitor or the like. In the present embodiment, the apparatus 10 for predicting change in a physical index is a dedicated device that predicts change in body weight. A display unit 11 and an input unit 12 are provided on the front face of the apparatus 10 for predicting change in a physical index. An acquisition unit 13 is provided on a side face of the apparatus 10 for predicting change in a physical index.

The display unit 11 can display a variety of images. For example, as described below, the display unit 11 can display an image indicating the current state of change in body weight, an image indicating the degree of increase in fat burning rate, and an image indicating the prediction of change in body weight.

The input unit 12 is, for example, configured using a plurality of button switches and detects a push by the user.

The acquisition unit 13 acquires the ketone body concentration excreted by a subject. A ketone body is a general term for acetoacetic acid, 3-hydroxybutyric acid ($\beta$-hydroxybutyric acid), and acetone and refers to at least one of these. In the present embodiment, an acetone sensor is used in the acquisition unit 13, and the ketone body concentration is acquired by detecting the acetone concentration included in the subject's breath. Note that while the acquisition unit 13 detects the acetone concentration included in breath in the present embodiment, alternatively the acquisition unit 13 may be configured to detect the ketone body concentration excreted by the subject through the skin, urine, saliva, sweat, or the like using a ketone body sensor other than an acetone sensor. In other words, the acquisition unit 13 may acquire the concentration of a ketone body other than acetone. Furthermore, the acquisition unit 13 may be configured to acquire, by communication or the like, the ketone body concentration detected by an external device.

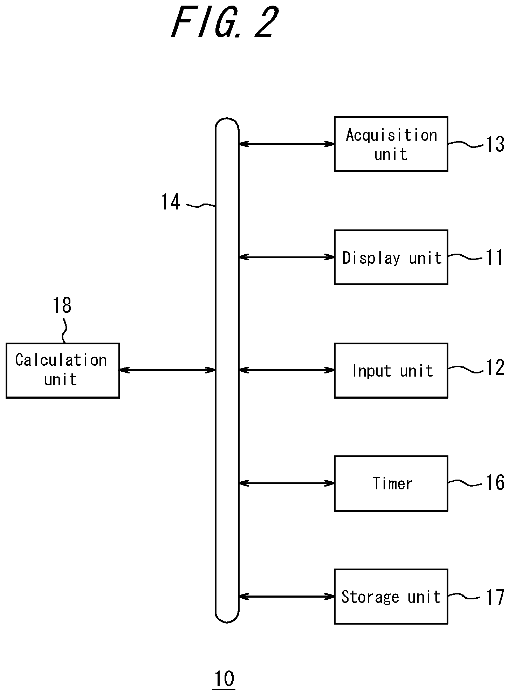

Next, the internal structure of the apparatus 10 for predicting change in a physical index is described using the functional block diagram in FIG. 2. The apparatus 10 for predicting change in a physical index includes the display unit 11, the input unit 12, the acquisition unit 13, a bus 14, a timer 16, a storage unit 17, and a calculation unit 18.

The bus 14 communicates information and control signals between the calculation unit 18, display unit 11, input unit 12, acquisition unit 13, timer 16, and storage unit 17.

The input unit 12 detects a variety of input to the apparatus 10 for predicting change in a physical index. For example, as described below, the input unit 12 detects input designating any time for which change in body weight is to be predicted. As described below, the input unit 12 also detects input of the subject's gender, Body Mass Index (BMI), and age; input to start acquisition of the ketone body concentration; input on whether to determine the current state of change in body weight; input on whether to determine the degree of increase in fat burning rate; and input on whether to predict change in body weight.

The timer 16 measures the current time.

The storage unit 17 is, for example, non-volatile semiconductor memory and stores the ketone body concentration acquired by the acquisition unit 13 in combination with the acquisition time, i.e. the current time measured by the timer 16 at the time of acquisition of the ketone body concentration. The storage unit 17 also stores the subject's gender, BMI, and age; a formula for calculating fat burning rate; a decision table for determining the current state of change in body weight; a decision table for determining the degree of increase in fat burning rate; a first formula for calculating a predicted value of change in body weight; a time threshold; a decision table for adjusting change in body weight; a difference threshold; a formula for calculating the coefficient of determination; and a second formula for calculating a predicted value of change in body weight.

The calculation unit 18 controls the operations of each unit in the apparatus 10 for predicting change in a physical index. For example, as described below, the calculation unit 18 makes the determination of the current state of change in body weight, the determination of the degree of increase in fat burning rate, and the prediction of change in body weight.

The determination of the current state of change in body weight by the apparatus 10 for predicting change in a physical index is now described. In the apparatus 10 for predicting change in a physical index, the determination of the current state of change in body weight is possible upon the acquisition unit 13 acquiring the ketone body concentration. Upon the acquisition unit 13 acquiring the ketone body concentration, the calculation unit 18 reads, from the storage unit 17, the formula for calculating fat burning rate shown in Formula (1) corresponding to the subject's stored gender.

$$Cal_{fat} = k_1 \times Conc + k_2 \qquad (1)$$

In Formula (1), Calfat is the fat burning rate (g/day), $k_1$ and $k_2$ are coefficients, and Conc is the acquired ketone body concentration (ppb). Formula (1) is calculated statistically as a regression formula that represents the relationship between ketone body concentration and fat burning rate, yielded by measuring the ketone body concentration and fat burning rate for multiple subjects and performing regression analysis on the measurement results. In the present embodiment, the formula for calculating fat burning rate is a first degree polynomial with respect to the ketone body concentration, yet the formula may be a polynomial of a different degree or a formula for the inverse, an index calculation, or a logarithmic calculation of the ketone body concentration.

Using the formula for calculating fat burning rate, the calculation unit 18 calculates the fat burning rate corresponding to the acquired ketone body concentration. Upon calculating the fat burning rate, the calculation unit 18 reads the decision table for determining the current state of change in body weight from the storage unit 17. This decision table determines the assessment grade, from grade 1 to grade 6, of the subject's current state of change in body weight. The assessment grades categorize the degree of the fat burning rate. As illustrated in Table 1, the current state of change in body weight is associated with one of the assessment grades in accordance with gender, age, BMI, and fat burning rate.

Table 1

| Gender | Age | BMI | Assessment grade corresponding to fat burning rate | | | | | |
| --- | --- | --- | --- | --- | --- | --- | --- | --- |
| | | | Grade 1 | Grade 2 | Grade 3 | Grade 4 | Grade 5 | Grade 6 |
| male | less than 20 | less than 18.5 | at least 0 and less than $a_{m1}$ | at least $a_{m1}$ and less than $b_{m1}$ | at least $b_{m1}$ and less than $c_{m1}$ | at least $c_{m1}$ and less than $d_{m1}$ | at least $d_{m1}$ and less than $e_{m1}$ | at least $e_{m1}$ |
| | | at least 18.5 and less than 22.0 | at least 0 and less than $a_{m2}$ | at least $a_{m2}$ and less than $b_{m2}$ | at least $b_{m2}$ and less than $c_{m2}$ | at least $c_{m2}$ and less than $d_{m2}$ | at least $d_{m2}$ and less than $e_{m2}$ | at least $e_{m2}$ |
| | | at least 22.0 and less than 25.0 | at least 0 and less than $a_{m3}$ | at least $a_{m3}$ and less than $b_{m3}$ | at least $b_{m3}$ and less than $c_{m3}$ | at least $c_{m3}$ and less than $d_{m3}$ | at least $d_{m3}$ and less than $e_{m3}$ | at least $e_{m3}$ |
| | | at least 25.0 | at least 0 and less than $a_{m4}$ | at least $a_{m4}$ and less than $b_{m4}$ | at least $b_{m4}$ and less than $c_{m4}$ | at least $c_{m4}$ and less than $d_{m4}$ | at least $d_{m4}$ and less than $e_{m4}$ | at least $e_{m4}$ |
| | at least 20 and less than 40 | less than 18.5 | at least 0 and less than $a_{m5}$ | at least $a_{m5}$ and less than $b_{m5}$ | at least $b_{m5}$ and less than $c_{m5}$ | at least $c_{m5}$ and less than $d_{m5}$ | at least $d_{m5}$ and less than $e_{m5}$ | at least $e_{m5}$ |
| | | at least 18.5 and less than 22.0 | at least 0 and less than $a_{m6}$ | at least $a_{m6}$ and less than $b_{m6}$ | at least $b_{m6}$ and less than $c_{m6}$ | at least $c_{m6}$ and less than $d_{m6}$ | at least $d_{m6}$ and less than $e_{m6}$ | at least $e_{m6}$ |
| | | at least 22.0 and less than 25.0 | at least 0 and less than $a_{m7}$ | at least $a_{m7}$ and less than $b_{m7}$ | at least $b_{m7}$ and less than $c_{m7}$ | at least $c_{m7}$ and less than $d_{m7}$ | at least $d_{m7}$ and less than $e_{m7}$ | at least $e_{m7}$ |
| | | at least 25.0 | at least 0 and less than $a_{m8}$ | at least $a_{m8}$ and less than $b_{m8}$ | at least $b_{m8}$ and less than $c_{m8}$ | at least $c_{m8}$ and less than $d_{m8}$ | at least $d_{m8}$ and less than $e_{m8}$ | at least $e_{m8}$ |

(continued)

| Gender | Age | BMI | Assessment grade corresponding to fat burning rate | | | | | |
|---|---|---|---|---|---|---|---|---|
| | | | Grade 1 | Grade 2 | Grade 3 | Grade 4 | Grade 5 | Grade 6 |
| | at least 40 and less than 60 | less than 18.5 | at least 0 and less than $a_{m9}$ | at least $a_{m9}$ and less than $b_{m9}$ | at least $b_{m9}$ and less than $c_{m9}$ | at least $c_{m9}$ and less than $d_{m9}$ | at least $d_{m9}$ and less than $e_{m9}$ | at least $e_{m9}$ |
| | | at least 18.5 and less than 22.0 | at least 0 and less than $a_{m10}$ | at least $a_{m10}$ and less than $b_{m10}$ | at least $b_{m10}$ and less than $c_{m10}$ | at least $c_{m10}$ and less than $d_{m10}$ | at least $d_{m10}$ and less than $e_{m10}$ | at least $e_{m10}$ |
| | | at least 22.0 and less than 25.0 | at least 0 and less than $a_{m11}$ | at least $a_{m11}$ and less than $b_{m11}$ | at least $b_{m11}$ and less than $c_{m11}$ | at least $c_{m11}$ and less than $d_{m11}$ | at least $d_{m11}$ and less than $e_{m11}$ | at least $e_{m11}$ |
| | | at least 25.0 | at least 0 and less than $a_{m12}$ | at least $a_{m12}$ and less than $b_{m12}$ | at least $b_{m12}$ and less than $c_{m12}$ | at least $c_{m12}$ and less than $d_{m12}$ | at least $d_{m12}$ and less than $e_{m12}$ | at least $e_{m12}$ |
| | at least 60 | less than 18.5 | at least 0 and less than $a_{m13}$ | at least $a_{m13}$ and less than $b_{m13}$ | at least $b_{m13}$ and less than $c_{m13}$ | at least $c_{m13}$ and less than $d_{m13}$ | at least $d_{m13}$ and less than $e_{m13}$ | at least $e_{m13}$ |
| | | at least 18.5 and less than 22.0 | at least 0 and less than $a_{m14}$ | at least $a_{m14}$ and less than $b_{m14}$ | at least $b_{m14}$ and less than $c_{m14}$ | at least $c_{m14}$ and less than $d_{m14}$ | at least $d_{m14}$ and less than $e_{m14}$ | at least $e_{m14}$ |
| | | at least 22.0 and less than 25.0 | at least 0 and less than $a_{m15}$ | at least $a_{m15}$ and less than $b_{m15}$ | at least $b_{m15}$ and less than $c_{m15}$ | at least $c_{m15}$ and less than $d_{m15}$ | at least $d_{m15}$ and less than $e_{m15}$ | at least $e_{m15}$ |
| | | at least 25.0 | at least 0 and less than $a_{m16}$ | at least $a_{m16}$ and less than $b_{m16}$ | at least $b_{m16}$ and less than $c_{m16}$ | at least $c_{m16}$ and less than $d_{m16}$ | at least $d_{m16}$ and less than $e_{m16}$ | at least $e_{m16}$ |
| female | less than 20 | less than 18.5 | at least 0 and less | at least $a_{f1}$ and less than | at least $b_{f1}$ and less than | at least $c_{f1}$ and less than | at least $d_{f1}$ and less than | at least $e_{f1}$ |

(continued)

| Gender | Age | BMI | Assessment grade corresponding to fat burning rate | | | | | |
|---|---|---|---|---|---|---|---|---|
| | | | Grade 1 | Grade 2 | Grade 3 | Grade 4 | Grade 5 | Grade 6 |
| | | | than $a_{f1}$ | $b_{f1}$ | $c_{f1}$ | $d_{f1}$ | $e_{f1}$ | |
| | | at least 18.5 and less than 22.0 | at least 0 and less than $a_{f2}$ | at least $a_{f2}$ and less than $b_{f2}$ | at least $b_{f2}$ and less than $c_{f2}$ | at least $c_{f2}$ and less than $d_{f2}$ | at least $d_{f2}$ and less than $e_{f2}$ | at least $e_{f2}$ |
| | | at least 22.0 and less than 25.0 | at least 0 and less than $a_{f3}$ | at least $a_{f3}$ and less than $b_{f3}$ | at least $b_{f3}$ and less than $c_{f3}$ | at least $c_{f3}$ and less than $d_{f3}$ | at least $d_{f3}$ and less than $e_{f3}$ | at least $e_{f3}$ |
| | | at least 25.0 | at least 0 and less than $a_{f4}$ | at least $a_{f4}$ and less than $b_{f4}$ | at least $b_{f4}$ and less than $c_{f4}$ | at least $c_{f4}$ and less than $d_{f4}$ | at least $d_{f4}$ and less than $e_{f4}$ | at least $e_{f4}$ |
| | at least 20 and less than 40 | less than 18.5 | at least 0 and less than $a_{f5}$ | at least $a_{f5}$ and less than $b_{f5}$ | at least $b_{f5}$ and less than $c_{f5}$ | at least $c_{f5}$ and less than $d_{f5}$ | at least $d_{f5}$ and less than $e_{f5}$ | at least $e_{f5}$ |
| | | at least 18.5 and less than 22.0 | at least 0 and less than $a_{f6}$ | at least $a_{f6}$ and less than $b_{f6}$ | at least $b_{f6}$ and less than $c_{f6}$ | at least $c_{f6}$ and less than $d_{f6}$ | at least $d_{f6}$ and less than $e_{f6}$ | at least $e_{f6}$ |
| | | at least 22.0 and less than 25.0 | at least 0 and less than $a_{f7}$ | at least $a_{f7}$ and less than $b_{f7}$ | at least $b_{f7}$ and less than $c_{f7}$ | at least $c_{f7}$ and less than $d_{f7}$ | at least $d_{f7}$ and less than $e_{f7}$ | at least $e_{f7}$ |
| | | at least 25.0 | at least 0 and less than $a_{f8}$ | at least $a_{f8}$ and less than $b_{f8}$ | at least $b_{f8}$ and less than $c_{f8}$ | at least $c_{f8}$ and less than $d_{f8}$ | at least $d_{f8}$ and less than $e_{f8}$ | at least $e_{f8}$ |
| | at least 40 and less than 60 | less than 18.5 | at least 0 and less than $a_{f9}$ | at least $a_{f9}$ and less than $b_{f9}$ | at least $b_{f9}$ and less than $c_{f9}$ | at least $c_{f9}$ and less than $d_{f9}$ | at least $d_{f9}$ and less than $e_{f9}$ | at least $e_{f9}$ |

(continued)

| Gender | Age | BMI | Assessment grade corresponding to fat burning rate | | | | | |
|---|---|---|---|---|---|---|---|---|
| | | | Grade 1 | Grade 2 | Grade 3 | Grade 4 | Grade 5 | Grade 6 |
| | | at least 18.5 and less than 22.0 | at least 0 and less than $a_{f10}$ | at least $a_{f10}$ and less than $b_{f10}$ | at least $b_{f10}$ and less than $c_{f10}$ | at least $c_{f10}$ and less than $d_{f10}$ | at least $d_{f10}$ and less than $e_{f10}$ | at least $e_{f10}$ |
| | | at least 22.0 and less than 25.0 | at least 0 and less than $a_{f11}$ | at least $a_{f11}$ and less than $b_{f11}$ | at least $b_{f11}$ and less than $c_{f11}$ | at least $c_{f11}$ and less than $d_{f11}$ | at least $d_{f11}$ and less than $e_{f11}$ | at least $e_{f11}$ |
| | | at least 25.0 | at least 0 and less than $a_{f12}$ | at least $a_{f12}$ and less than $b_{f12}$ | at least $b_{f12}$ and less than $c_{f12}$ | at least $c_{f12}$ and less than $d_{f12}$ | at least $d_{f12}$ and less than $e_{f12}$ | at least $e_{f12}$ |
| | at least 60 | less than 18.5 | at least 0 and less than $a_{f13}$ | at least $a_{f13}$ and less than $b_{f13}$ | at least $b_{f13}$ and less than $c_{f13}$ | at least $c_{f13}$ and less than $d_{f13}$ | at least $d_{f13}$ and less than $e_{f13}$ | at least $e_{f13}$ |
| | | at least 18.5 and less than 22.0 | at least 0 and less than $a_{f14}$ | at least $a_{f14}$ and less than $b_{f14}$ | at least $b_{f14}$ and less than $c_{f14}$ | at least $c_{f14}$ and less than $d_{f14}$ | at least $d_{f14}$ and less than $e_{f14}$ | at least $e_{f14}$ |
| | | at least 22.0 and less than 25.0 | at least 0 and less than $a_{f15}$ | at least $a_{f15}$ and less than $b_{f15}$ | at least $b_{f15}$ and less than $c_{f15}$ | at least $c_{f15}$ and less than $d_{f15}$ | at least $d_{f15}$ and less than $e_{f15}$ | at least $e_{f15}$ |
| | | at least 25.0 | at least 0 and less than $a_{f16}$ | at least $a_{f16}$ and less than $b_{f16}$ | at least $b_{f16}$ and less than $c_{f16}$ | at least $c_{f16}$ and less than $d_{f16}$ | at least $d_{f16}$ and less than $e_{f16}$ | at least $e_{f16}$ |

Based on the fat burning rate for the subject's stored gender, age, and BMI, the calculation unit 18 selects one of the assessment grades in the read decision table for determining the current state of change in body weight. In the present embodiment, the assessment grades are categorized from grade 1 to grade 6, yet any plural number of assessment grades may be used. Fat burning is extremely low at grade 1 and increases from grade 1 to grade 6.

Note that the thresholds for classifying the assessment grades in Table 1 ($a_{m1}$ to $a_{m16}$, $b_{m1}$ to $b_{m16}$, $c_{m1}$ to $c_{m16}$, $d_{m1}$ to $d_{m16}$, $e_{m1}$ to $e_{m16}$, $a_{f1}$ to $a_{f16}$, $b_{f1}$ to $b_{f16}$, $c_{f1}$ to $c_{f16}$, $d_{f1}$ to $d_{f16}$, $e_{f1}$ to $e_{f16}$) are prepared in advance by a procedure such as the following. The gender, age, and BMI are collected for multiple subjects. Next, for a variety of circumstances for each subject (for example, a variety of lengths of time after a meal and a variety of exercise intensities), the fat burning rate is calculated along with the actual change in body weight, and an observation of physical condition is made by a physician, nutrition care manager, or the like. Based on the actual change in body weight and the observation of physical condition, the physician, nutrition care manager, or the like classifies the change in body weight of each subject into one of the above grades 1 through 6 for each of the variety of circumstances. Each classified assessment grade is associated with the fat burning rate calculated for the same circumstances. For the multiple subjects, a distribution chart of the sample size is created to show the fat burning rate at each gender, age, BMI, and assessment grade. The thresholds separating adjacent assessment grades, such as grade 1 and grade 2, are determined based on this distribution chart. Note that while BMI is used in the distribution chart for determining the current state of change in body weight, a different index indicating body size or degree of build may be used. For example, body surface area, body weight, body fat percentage, amount of body fat, or amount of body fat normalized by height may be used. Furthermore, a combination of these indices may be used.

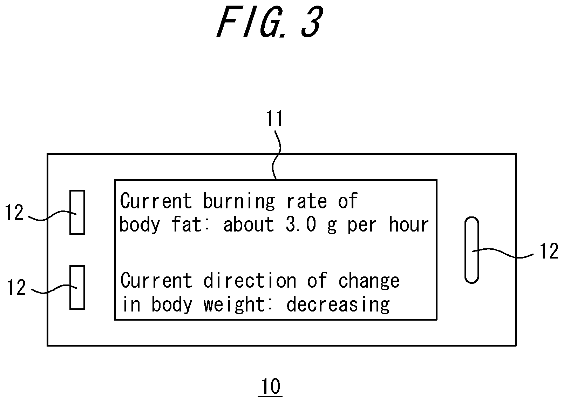

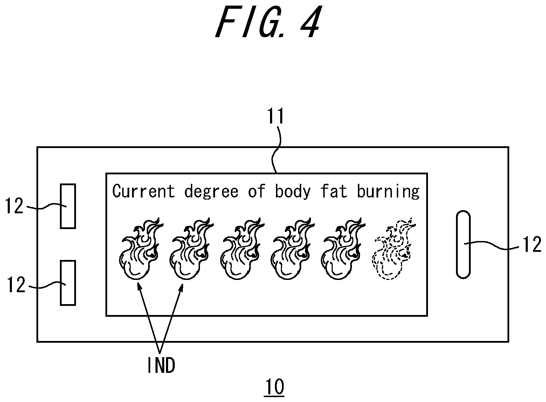

Upon selecting the assessment grade corresponding to the fat burning rate, the calculation unit 18 creates an image indicating the current state of change in body weight. The image indicating the current state of change in body weight includes, for example, a value yielded by converting the calculated fat burning rate per day into a rate per hour as well as the direction of change in body weight in accordance with the assessment grade, as illustrated in FIG. 3. The direction in accordance with the assessment grade is, for example, determined to be increasing for grade 1, steady for grade 2 or 3, and decreasing for grade 4, 5, or 6 in the decision table for determining the current state of change in body weight (Table 1). In the example in FIG. 3, the direction of change in body weight is shown for grade 5. The image indicating the current state of change in body weight may, for example, display a number of indicators IND corresponding to the assessment grade, as illustrated in FIG. 4, to provide a sensory perception of the current state of change in body weight. In the example in FIG. 4, grade 5 is shown by the indicators IND.

The determination of degree of increase in fat burning rate is now described. In the apparatus 10 for predicting change in a physical index, the determination of the degree of increase in fat burning rate is possible upon the acquisition unit 13 acquiring a new ketone body concentration in a state when the storage unit 17 is storing a ketone body concentration acquired in the past. Upon the acquisition unit 13 acquiring the new ketone body concentration, the calculation unit 18 reads the ketone body concentration stored in combination with the most recent acquisition time from the storage unit 17. The calculation unit 18 also reads the decision table for determining the degree of increase in fat burning rate from the storage unit 17. This decision table assigns the degree of fat burning of the subject during the time period from the last measurement to the current measurement of ketone body concentration to one of the following categories of degree of increase (decrease) in fat burning rate: down, stable, speed up, super speed up, and overexertion warning. As illustrated in Table 2, one of the degrees of increase in fat burning rate is assigned in accordance with age, past ketone body concentration, and a comparison of the newly acquired ketone body concentration with the read ketone body concentration.

Table 2

| Age | Past ketone body concentration | Categories of degree of increase in fat burning rate corresponding to comparison of the new and past ketone body concentrations | | | | |
|---|---|---|---|---|---|---|
| | | down | stable | speed up | super speed up | overexertion warning |
| less than 20 | less than 200 | $curConc_{ktn} < k_3 \times preConc_{ktn}$ | $1 \leq \Delta Conc < f_1$ | $f_1 \leq \Delta Conc < g_1$ | $g_1 \leq \Delta Conc < h_1$ | $h_1 \leq \Delta Conc$ |
| | at least 200 and less than 500 | | $1 \leq \Delta Conc < f_2$ | $f_2 \leq \Delta Conc < g_2$ | $g2 \leq \Delta Conc < h_2$ | $h_2 \leq \Delta Conc$ |
| | at least 500 and less than 800 | | $1 \leq \Delta Conc < f_3$ | $f_3 \leq \Delta Conc < g_3$ | $g_3 \leq \Delta Conc < h_3$ | $h_3 \leq \Delta Conc$ |

(continued)

| Age | Past ketone body concentration | Categories of degree of increase in fat burning rate corresponding to comparison of the new and past ketone body concentrations | | | | |
|---|---|---|---|---|---|---|
| | | down | stable | speed up | super speed up | overexertion warning |
| | at least 800 | | $1 \leq \Delta Conc < f_4$ | $f_4 \leq \Delta Conc < g_4$ | $g_4 \leq \Delta Conc < h_4$ | $h_4 \leq \Delta Conc$ |
| at least 20 and less than 40 | less than 200 | | $1 \leq \Delta Conc < f_5$ | $f_5 \leq \Delta Conc < g_5$ | $g_5 \leq \Delta Conc < h_5$ | $h_5 \leq \Delta Conc$ |
| | at least 200 and less than 500 | | $1 \leq \Delta Conc < f_6$ | $f_6 \leq \Delta Conc < g_6$ | $g_6 \leq \Delta Conc < h_6$ | $h_6 \leq \Delta Conc$ |
| | at least 500 and less than 800 | | $1 \leq \Delta Conc < f_7$ | $f_7 \leq \Delta Conc < g_7$ | $g_7 \leq \Delta Conc < h_7$ | $h_7 \leq \Delta Conc$ |
| | at least 800 | | $1 \leq \Delta Conc < f_8$ | $f_8 \leq \Delta Conc < g_8$ | $g_8 \leq \Delta Conc < h_8$ | $h_8 \leq \Delta Conc$ |
| at least 40 and less than 60 | less than 200 | | $1 \leq \Delta Conc < f_9$ | $f_9 \leq \Delta Conc \leq g_9$ | $g_9 \leq \Delta Conc < h_9$ | $h_9 \leq \Delta Conc$ |
| | at least 200 and less than 500 | | $1 \leq \Delta Conc < f_{10}$ | $f_{10} \leq \Delta Conc < g_{10}$ | $g_{10} \leq \Delta Conc < hio$ | $h_{10} \leq \Delta Conc$ |
| | at least 500 and less than 800 | | $1 \leq \Delta Conc < f_{11}$ | $f_{11} \leq \Delta Conc < g_{11}$ | $g_{11} \leq \Delta Conc < h_{11}$ | $h_{11} \leq \Delta Conc$ |
| | at least 800 | | $1 \leq \Delta Conc < f_{12}$ | $f_{12} \leq \Delta Conc < g_{12}$ | $g_{12} \leq \Delta Conc < h_{12}$ | $h_{12} \leq \Delta Conc$ |
| at least 60 | less than 200 | | $1 \leq \Delta Conc < f_{13}$ | $f_{13} \leq \Delta Conc < g_{13}$ | $g_{13} \leq \Delta Conc < h_{13}$ | $h_{13} \leq \Delta Conc$ |
| | at least 200 and less than 500 | | $1 \leq \Delta Conc < f_{14}$ | $f_{14} \leq \Delta Conc < g_{14}$ | $g_{14} \leq \Delta Conc < h_{14}$ | $h_{14} \leq \Delta Conc$ |
| | at least 500 and less than 800 | | $1 \leq \Delta Conc < f_{15}$ | $f_{15} \leq \Delta Conc < g_{15}$ | $g_{15} \leq \Delta Conc < h_{15}$ | $h_{15} \leq \Delta Conc$ |
| | at least 800 | | $1 \leq \Delta Conc < f_{16}$ | $f_{16} \leq \Delta Conc \leq g_{16}$ | $g_{16} \leq \Delta Conc < h_{16}$ | $h_{16} \leq \Delta Conc$ |

In Table 2, $k_3$ is a freely chosen coefficient less than one, and $curConc_{ktn}$ is the ketone body concentration newly acquired by the acquisition unit 13. Furthermore, $preConc_{ktn}$ is the past ketone body concentration read from the storage unit 17, and $\Delta Conc$ is the result of subtracting the past ketone body concentration from the newly acquired ketone body concentration, i.e. ($curConc_{ktn}$ - $preConc_{ktn}$). The thresholds for determining the degree of increase in fat burning rate ($f_1$ to $f_{16}$, $g_1$ to $g_{16}$, $h_1$ to $h_{16}$) are set to decrease with increasing age. For example, when the past ketone body concentration is less than 200, the thresholds $f_1$, $f_5$, $f_9$, and $f_{13}$ that are the upper limit on the degree of increase in fat burning rate indicating "stable" satisfy the relationships $f_1 > f_5 > f_9 > f_{13}$. Furthermore, the thresholds for determining the degree of increase in fat burning rate ($f_1$ to $f_{16}$, $g_1$ to $g_{16}$, $h_1$ to $h_{16}$) are set to increase as the past ketone body concentration grows larger. For example, for an age of less than 20, the thresholds $f_1$, $f_2$, $f_3$, and $f_4$ that are the upper limit on the degree of increase in fat burning rate indicating "stable" satisfy the relationships $f_1 < f_2 < f_3 < f_4$.

Table 2 is created by collecting the age, a ketone body concentration acquired in the past, and a newly acquired ketone body concentration for multiple subjects, classifying the degree of fat burning of each subject into one of the categories of down, stable, speed up, super speed up, and overexertion warning, and based on a distribution of the age, the past ketone body concentration, and a comparison of new and past ketone body concentrations in each category, the thresholds ($f_1$ to $f_{16}$, $g_1$ to $g_{16}$, $h_1$ to $h_{16}$) are set as boundaries for each category.

Based on the subject's stored age and amount of change in ketone body concentration, the calculation unit 18 selects one of the categories of degree of increase in fat burning rate from the read decision table for determining the degree of increase in fat burning rate. In the present embodiment, the degree of increase in fat burning rate is divided into five grades, i.e. "down", "stable", "speed up", "super speed up", and "overexertion warning", yet any plural number of grades may be used. Furthermore, in the present embodiment, in the decision table for determining the degree of increase in fat burning rate, the thresholds for determination are established in correspondence with age and past ketone body concentration, yet thresholds corresponding to at least one of age and past ketone body concentration need not be established. Alternatively, thresholds for determination may be established in correspondence with gender or a physical attribute such as BMI.

Upon selecting the current degree of increase in fat burning rate, the calculation unit 18 calculates the length of time from the acquisition time of the past ketone body concentration used to select the degree of increase in fat burning rate until the current time measured by the timer 16. Furthermore, the calculation unit 18 determines whether the length of time exceeds the time threshold. The time threshold is a value established to determine whether the length of time is appropriate for selection of the degree of increase in fat burning rate. Lengths of time are collected for multiple subjects, the degree of increase in fat burning rate is calculated, and it is determined whether the status of fat burning for each subject corresponds to the degree of increase in fat burning rate. Based on a distribution of the lengths of time of subjects for which this determination is made, the time threshold is established.

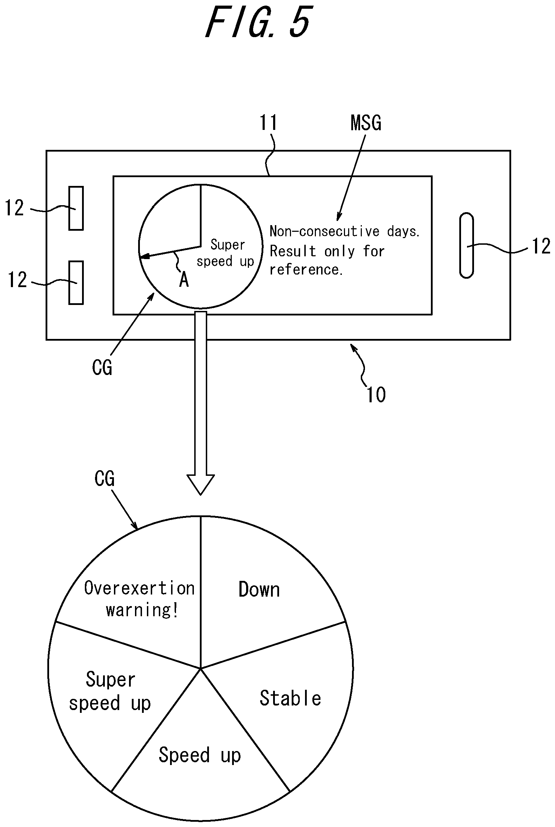

Next, the calculation unit 18 creates an image indicating the degree of increase in fat burning rate. The image indicating the degree of increase in fat burning rate displays, for example, the current degree of increase in fat burning rate with a circular graph CG as illustrated in FIG. 5. In the circular graph CG, as the angle of rotation of the arrow A increases from a direction pointing straight up, the indication changes between regions showing that the degree of increase in fat burning rate is "down", "stable", "speed up", "super speed up", and "overexertion warning". When the length of time exceeds the time threshold, the image indicating the degree of increase in fat burning rate displays a message MSG indicating that the displayed degree of increase in fat burning rate is a determination for reference, such as "Non-consecutive days. Result only for reference", "Comparison with three or more days ago. Result only for reference", "Too many days since comparison data. Result only for reference" or the like.

The prediction of change in body weight is now described. In the apparatus 10 for predicting change in a physical index, the prediction of change in body weight is possible upon the acquisition unit 13 acquiring the ketone body concentration. Upon the acquisition unit 13 acquiring the ketone body concentration, the input unit 12 becomes capable of detecting input designating any time for which change in body weight is to be predicted. Upon the input unit 12 detecting the designated time, the calculation unit 18 calculates the length of time from the current time measured by the timer 16 until the designated time. Upon calculating the length of time, the calculation unit 18 reads the first formula for calculating a predicted value of change in body weight, shown by Formula (2), from the storage unit 17.

$$\Delta W = k_4 \times \left( \Delta t \times Conc \right)^2 + k_5 \times \left( \Delta t \times Conc \right) + k_6 \qquad (2)$$

In Formula (2), $\Delta W$ is the predicted value of change in body weight, $k_4$, $k_5$, and $k_6$ are coefficients, and $\Delta t$ is the length of time. Formula (2) is calculated statistically as a regression formula that represents the relationship between ketone body concentration, length of time, and change in body weight, yielded by measuring the ketone body concentration and length of time for multiple subjects and performing regression analysis on the measurement results. In the present embodiment, the first formula for calculating a predicted value of change in body weight is a second degree polynomial for the product of the length of time and the ketone body concentration, as illustrated in Formula (2), yet the formula may be a polynomial of a different degree or a formula including either or both of the length of time and the ketone body concentration as separate terms. Furthermore, the first formula for calculating a predicted value of change in body weight may be a formula for the inverse, an index calculation, or a logarithmic calculation of the length of time and the ketone body concentration.

Using the first formula for calculating a predicted value of change in body weight, the calculation unit 18 calculates the predicted value of change in body weight of the subject (predicted value of change in the physical index) for the calculated length of time and the newly acquired ketone body concentration.

When the storage unit 17 has not stored a past ketone body concentration, the calculated predicted value is treated as the final predicted value. Conversely, when the storage unit 17 has stored a past ketone body concentration, the calculation unit 18 treats the predicted value calculated as described above as a temporary predicted value, and based on the past ketone body concentration, calculates the final predicted value.

When the storage unit 17 has stored a single past ketone body concentration, the calculation unit 18 reads, from the storage unit 17, the past ketone body concentration, the acquisition time, and the decision table for adjusting change in body weight. This decision table is for determining an adjustment coefficient used to calculate the final predicted value. As illustrated in Table 3, each category of the adjustment coefficient is listed in correspondence with a degree of increase in fat burning rate. The degree of increase in fat burning rate referred to here is the degree of increase in fat burning rate selected when determining the degree of increase in fat burning rate as described above.

Table 3

|  | down | stable | speed up | super speed up | overexertion warning |
|---|---|---|---|---|---|
| Adjustment coefficient | $k_7$ | no adjustment | $k_8$ | $k_9$ | $k_{10}$ |

In Table 3, the coefficients are established so that $-1 < k_7 < 0 < k_8 < k_9 < k_{10} < 1$. The calculation unit 18 selects the degree of increase in fat burning rate as described above and then selects one of the adjustment coefficients from the read decision table for adjusting change in body weight in accordance with the adjustment coefficient corresponding to the determined degree of increase in fat burning rate.

Upon selecting the adjustment coefficient, the calculation unit 18 uses the temporary predicted value of change in body weight calculated by Formula (2) and the selected adjustment coefficient to calculate the final predicted value of change in body weight. The formula for calculating the final predicted value of change in body weight may be any formula that can correct the temporary predicted value in accordance with the selected adjustment coefficient. For example, the final predicted value may be calculated using any of Formulas (3) through (7).

$$\Delta W_{fin} = \Delta W \times \left(1 + k_{slct}\right) \tag{3}$$

$$\Delta W_{fin} = \Delta W + k_{slct}\left(curConc_{ktn} - preConc_{ktn}\right) \tag{4}$$

$$\Delta W_{fin} = \Delta W \times k_{slct}\left(curConc_{ktn} - preConc_{ktn}\right) \tag{5}$$

$$\Delta W_{fin} = \Delta W + k_{slct} \times \frac{curConc_{ktn}}{preConc_{ken}} \tag{6}$$

$$\Delta W_{fin} = \Delta W \times k_{slct} \times \frac{curConc_{ktn}}{preConc_{ken}} \tag{7}$$

In Formulas (3) through (7), $\Delta W_{fin}$, is the final predicted value of change in body weight, and $k_{slct}$ is the adjustment coefficient $k_7$ through $k_{10}$ selected by the decision table for adjusting change in body weight (Table 3).

The adjustment coefficients $k_7$ through $k_{10}$ in Table 3 are calculated as follows. The change in body weight after each length of time is measured for multiple subjects, the predicted value of change in body weight is calculated using the first formula for calculating a predicted value of change in body weight (Formula (2)), and the degree of increase in fat burning rate is selected. For each degree of increase in fat burning rate, a regression analysis is performed on the relationship between the measured change in body weight and the calculated predicted value of change in body weight, and the adjustment coefficients are calculated statistically as coefficients used in regression formulas that represent the relationship of the predicted value of change in body weight to the measured change in body weight and the relationship of the predicted value of change in body weight, the past ketone body concentration, and the new ketone body concentration to the measured change in body weight.

When the storage unit 17 has stored a plurality of past ketone body concentrations, the calculation unit 18 reads, from the storage unit 17, the past ketone body concentrations and the acquisition times. The calculation unit 18 then performs a regression analysis on the ketone body concentrations for the read acquisition times and creates a temporary formula for calculating the ketone body concentration at any designated time, as shown illustrated in Formula (8).

$$estConc = k_{11} \times t + k_{12} \tag{8}$$

In Formula (8), estConc is the temporary predicted value of the ketone body concentration at time t. The coefficients $k_{11}$ and $k_{12}$ are established by the above-described regression analysis. In the present embodiment, the temporary formula for calculating the ketone body concentration is a linear regression formula by first-order approximation, as illustrated in Formula (8), yet a curve approximation may be used.

Using the temporary formula for calculating the ketone body concentration, i.e. Formula (8), the calculation unit 18 calculates the temporary predicted value of the ketone body concentration at the acquisition time of the actually acquired past ketone body concentration. The calculation unit 18 then calculates the difference between the ketone body concentration acquired at the same acquisition time and the temporary predicted value. The calculation unit 18 reads the difference threshold from the storage unit 17 and compares the difference threshold with the difference between the acquired ketone body concentration and the temporary predicted value. The calculation unit 18 then excludes the ketone body concentration at an acquisition time for which the difference is greater than the difference threshold. The difference threshold is a value established for determining whether to exclude an acquired ketone body concentration as being unsuitable for regression analysis in the formula for final calculation of the ketone body concentration. The difference threshold is established so that the difference between the predicted value of change in body weight, calculated with the below-described second formula for calculating a predicted value of change in body weight, and the measured change in body weight is less than a predetermined value.

The calculation unit 18 performs regression analysis on the ketone body concentrations for the remaining acquisition times that were not excluded and creates a formula for final calculation of the ketone body concentration similar to Formula (8), i.e. a linear regression formula by first-order approximation. Using the formula for final calculation, the calculation unit 18 calculates the predicted value of the ketone body concentration for a date and time immediately before the date and time of prediction (for example, one hour before). The calculation unit 18 then substitutes the length of time and the calculated predicted value of the ketone body concentration into the first formula for calculating a predicted value of change in body weight (Formula (2)) in order to calculate the predicted value of change in body weight based on the predicted ketone body concentration.

Next, from the storage unit 17, the calculation unit 18 reads the formula for calculating the coefficient of determination such as Formula (9).

$$r^2 = \frac{\sum \left( estConc_i - estConc_{ave} \right)^2}{\sum \left( preConc_i - preConc_{ave} \right)^2} \qquad (9)$$

In Formula (9), r represents the coefficient of determination, est $Conc_i$ represents the final predicted value of each ketone body concentration, est $Conc_{ave}$ represents the average of the final predicted values of the ketone body concentration, $preConc_i$ represents the ketone body concentrations remaining without being excluded, and $preConc_{ave}$ represents the average of the ketone body concentrations remaining without being excluded.

Using the formula for calculating the coefficient of determination, the calculation unit 18 calculates the coefficient of determination for the final predicted value of each ketone body concentration and for each ketone body concentration remaining without being excluded. From the storage unit 17, the calculation unit 18 also reads the second formula for calculating a predicted value of change in body weight such as Formula (10).

$$\Delta W_{fin} = k_{13} \times temp\Delta W + \left( 1 - k_{13} \right) \times est\Delta W \qquad (10)$$

In Formula (10), $k_{13}$ is a coefficient of 1 or less established based on the coefficient of determination, such as $r^2$. Furthermore, $temp\Delta W$ is the temporary predicted value based on the newly acquired ketone body concentration and is calculated using the first formula for calculating a predicted value of change in body weight. The term est $\Delta W$ is the predicted value of change in body weight based on the final predicted value of the ketone body concentration and is calculated using the first formula for calculation. Accordingly, Formula (10) calculates a weighted average of the temporary predicted value based on the newly acquired ketone body concentration and the final predicted value of the ketone body concentration calculated using the first formula for calculation.

Using the second formula for calculating a predicted value of change in body weight, the calculation unit 18 calculates the final predicted value of change in body weight based on the following: the coefficient calculated based on the coefficient of determination, the temporary predicted value based on the newly acquired ketone body concentration, and the predicted value of the ketone body concentration.

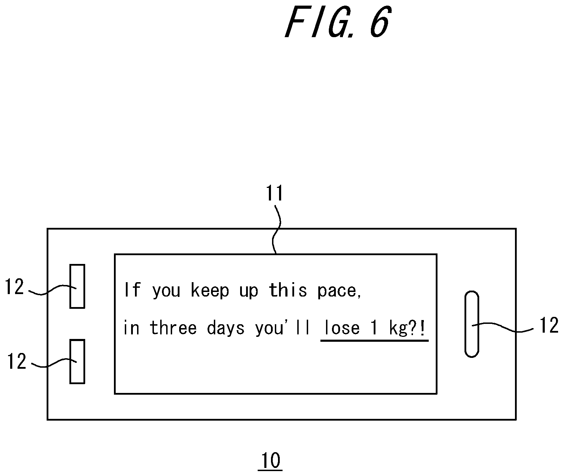

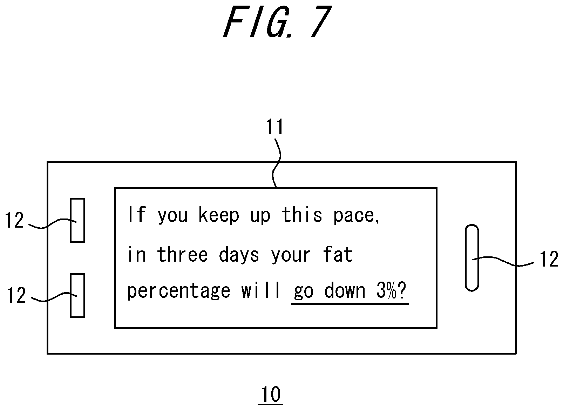

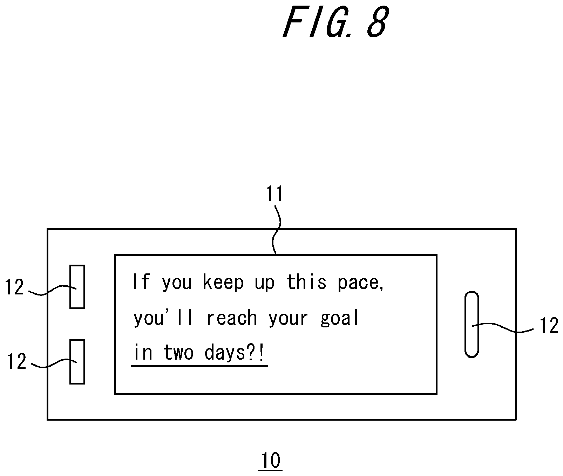

Upon calculating the final predicted value of change in body weight, the calculation unit 18 creates an image indicating prediction of change in body weight. The image indicating prediction of change in body weight displays, for example, the final predicted value of change in body weight at the designated time detected by the input unit 12, as illustrated in FIG. 6. Alternatively, the image indicating prediction of change in body weight may, for example, display a predicted value of change in body fat percentage corresponding to the change in body weight, as illustrated in FIG. 7. If a target value for change in body weight is input into the input unit 12, the time at which the target will be achieved may be displayed in the image indicating prediction of change in body weight (see FIG. 8).

Furthermore, in accordance with the predicted value of change in body weight, the calculation unit 18 may create images indicating advice for meals, exercise, and lifestyle, as well as related information, and cause the display unit 11 to display the images. The calculation unit 18 may also create a graph illustrating the change in body weight until the designated time detected by the input unit 12 and cause the display unit 11 to display the graph.

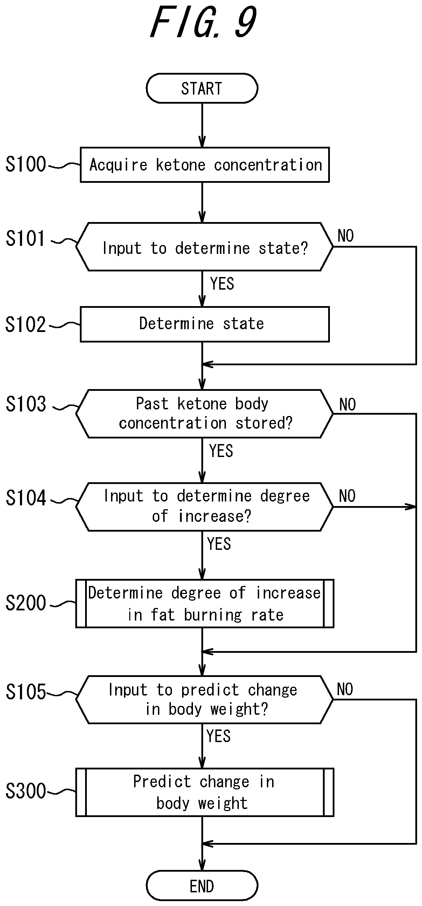

Next, the processing executed by the calculation unit 18 to observe change in body weight is described using the flowchart in FIG. 9. The processing to observe change in body weight is processing, in the apparatus 10 for predicting change in a physical index, to determine the current state of change in body weight, to determine the degree of increase in fat burning rate, and to predict change in body weight. The processing to observe change in body weight begins when the input unit 12 detects input to start acquisition of the ketone body concentration.

In step S100, the calculation unit 18 causes the acquisition unit 13 to acquire the ketone body concentration. Upon acquisition of the ketone body concentration, processing proceeds to step S101.

In step S101, the calculation unit 18 decides whether input to determine the current state of change in body weight is provided. When detecting such input, processing proceeds to step S102. When such input is not detected, processing skips step S102 and proceeds to step S103.

In step S102, the calculation unit 18 determines the current state of change in body weight. In other words, the calculation unit 18 reads the formula for calculating fat burning rate (Formula (1)) corresponding to the subject's gender from the storage unit 17 and calculates the fat burning rate corresponding to the ketone body concentration acquired in step S100. The calculation unit 18 also reads the decision table for determining the current state of change in body weight (Table 1) from the storage unit 17 and selects an assessment grade based on the calculated fat burning rate. Furthermore, using at least one of the calculated fat burning rate and the selected assessment grade, the calculation unit 18 creates an image indicating the current state of change in body weight and causes the display unit 11 to display the image. Upon completion of the above determination of the current state of change in body weight, processing proceeds step S103.

In step S103, the calculation unit 18 determines whether a ketone body concentration acquired in the past is stored in the storage unit 17. When such a ketone body concentration is stored, processing proceeds to step S104. Otherwise, processing skips steps S104 and S200 and proceeds to step S105.

In step S104, the calculation unit 18 decides whether input to determine the degree of increase in fat burning rate is provided. When detecting such input, processing proceeds to step S200. When such input is not detected, processing skips step S200 and proceeds to step S105.

In step S200, as described below, a subroutine for determining the degree of increase in fat burning rate is executed. Upon causing the display unit 11 to display an image indicating the degree of increase in fat burning rate by executing the subroutine for determining the degree of increase in fat burning rate, processing proceeds to step S105.

In step S105, the calculation unit 18 decides whether input to predict change in body weight is provided, and when detecting such input, processing proceeds to step S300. When such input is not detected, the ketone body concentration acquired in step S100 is stored in the storage unit 17 in combination with the current time, and processing to observe change in body weight terminates.

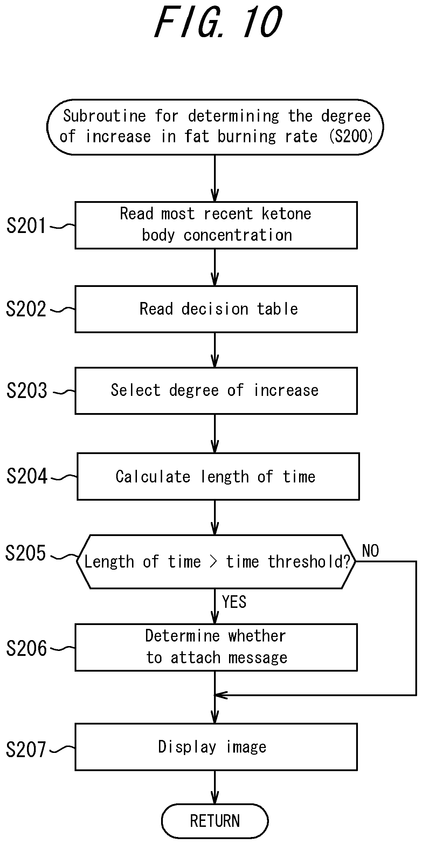

Next, the subroutine for determining the degree of increase in fat burning rate executed by the calculation unit 18 in step S200 is described using the flowchart in FIG. 10.

In step S201, the calculation unit 18 reads the ketone body concentration with the most recent acquisition time from the storage unit 17. Upon reading of the ketone body concentration, processing proceeds to step S202.

In step S202, the calculation unit 18 reads the decision table for determining the degree of increase in fat burning rate (Table 2). Upon reading of the decision table, processing proceeds to step S203.

In step S203, the calculation unit 18 selects one of the degrees of increase in fat burning rate in the decision table read in step S203 based on a comparison of the ketone body concentration acquired in step S100 and the ketone body concentration read in step S201. Upon selection of the degree of increase in fat burning rate, processing proceeds to step S204.

In step S204, the calculation unit 18 calculates the length of time from the acquisition time of the ketone body concentration read in step S201 until the current time measured by the timer 16. Upon calculation of the length of time, processing proceeds to step S205.

In step S205, the calculation unit 18 determines whether the length of time calculated in step S204 is greater than the time threshold read from the storage unit 17. When the length of time is greater than the time threshold, processing proceeds to step S206. When the length of time is equal to or less than the time threshold, processing skips step S206 and proceeds to step S207.

In step S206, the calculation unit 18 determines whether to attach a message to the created image indicating that the result is only for reference. After determining whether to attach the message, processing proceeds to step S208.

In step S207, the calculation unit 18 creates an image indicating the degree of increase in fat burning rate based on the degree of increase in fat burning rate determined in step S204 and on the message when, in step S207, it is determined to attach the message. The calculation unit 18 then causes the display unit 11 to display the image.

After display of the image indicating the degree of increase in fat burning rate, the subroutine for determining the degree of increase in fat burning rate terminates.

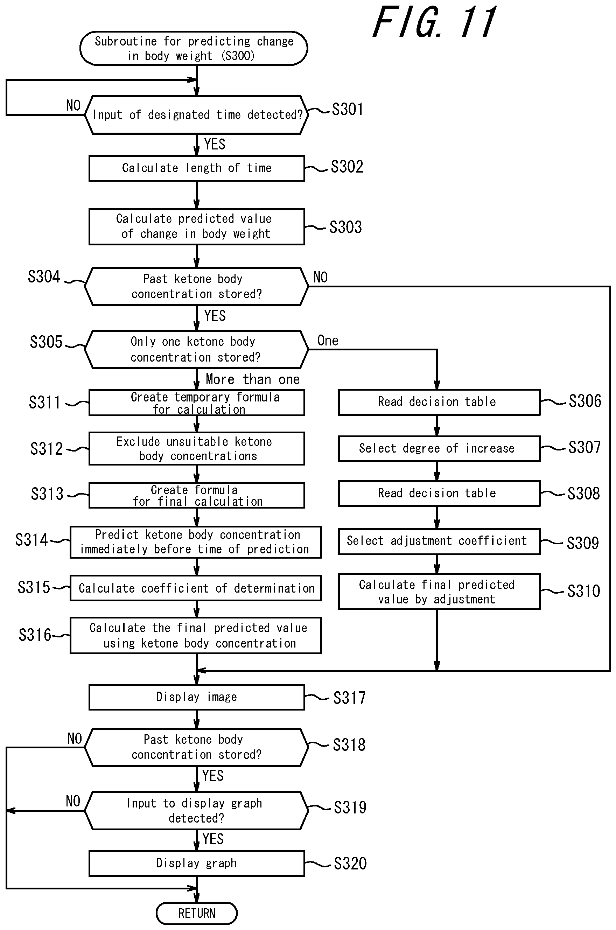

Next, the subroutine for predicting change in body weight executed by the calculation unit 18 in step S300 is described using the flowchart in FIG. 11.

In step S301, the calculation unit 18 determines whether the input unit 12 has detected input of a designated time. When input of a designated time has not been detected, step S301 is repeated. When input of a designated time has been detected, processing proceeds to step S302.

In step S302, the calculation unit 18 calculates the length of time from the current time measured by the timer 16 until the designated time detected in step S301. Upon finishing calculation of the length of time, processing proceeds to step S303.

In step S303, using the first formula for calculating a predicted value of change in body weight (Formula (2)) stored in the storage unit 17, the calculation unit 18 calculates the predicted value of change in body weight after the length of time calculated in step S302. Upon calculation of the predicted value of change in body weight, processing proceeds to step S304.

In step S304, the calculation unit 18 determines whether a ketone body concentration acquired in the past is stored in the storage unit 17. When such a ketone body concentration is stored, processing proceeds to step S305. Otherwise, processing proceeds to step S318.

In step S305, the calculation unit 18 determines whether only one ketone body concentration is stored in the storage unit 17. When one ketone body concentration is stored, processing proceeds to step S306. When a plurality of ketone body concentrations is stored, processing proceeds to step S312.

In steps S306 and S307, the calculation unit 18 executes the same operations as in steps S202 and S203 of the subroutine for determining the degree of increase in fat burning rate. Upon selection of the degree of increase in fat burning rate in step S307, processing proceeds to step S308.

In step S308, the calculation unit 18 reads the decision table for determining the degree of increase in fat burning rate (Table 3) from the storage unit 17. Upon reading of the decision table, processing proceeds to step S309.

In step S309, based on the degree of increase in fat burning rate selected in step S308, the calculation unit 18 selects one of the adjustment coefficients in the decision table (Table 3) read in step S309. Upon determination of the adjustment coefficient, processing proceeds to step S310.

In step S310, the calculation unit 18 calculates the final predicted value of change in body weight by adjusting the predicted value of change in body weight calculated in step S303 using the adjustment coefficient selected in step S309 (Formula (3) through Formula (7)). Upon calculation of the final predicted value, processing proceeds to step S317.

As described above, in the case of a plurality of ketone body concentrations in step S305, processing proceeds to step S311. In step S311, the calculation unit 18 performs regression analysis based on the past ketone body concentrations and acquisition times read from the storage unit 17 and creates a temporary formula for calculating the ketone body concentration (Formula (8)). Upon creation of the temporary formula for calculation, processing proceeds to step S312.

In step S312, the calculation unit 18 excludes ketone body concentrations for which the difference from the ketone body concentration calculated using the temporary formula for calculation (Formula (8)) calculated in step S311 is greater than the difference threshold. Upon exclusion of any unsuitable ketone body concentrations, processing proceeds to step S313.

In step S313, the calculation unit 18 performs regression analysis based on the ketone body concentrations remaining after exclusion in step S312 and the corresponding acquisition times and creates a formula for final calculation of the ketone body concentration. Upon creation of the formula for final calculation, processing proceeds to step S314.

In step S314, using the formula for final calculation created in step S313, the calculation unit 18 calculates the predicted value of the ketone body concentration for a date and time immediately before the date and time of prediction (for example, one hour before). Upon calculation of the predicted value, processing proceeds to step S315.

In step S315, using the formula for calculating the coefficient of determination (Formula (9)), the calculation unit 18 calculates the coefficient of determination for the ketone body concentration based on the formula for final calculation created in step S313 and for ketone body concentrations remaining after exclusion in step S312. Upon calculation of the coefficient of determination, processing proceeds to step S316.

In step S316, using the second formula for calculating a predicted value of change in body weight (Formula (10)) stored in the storage unit 17, the calculation unit 18 calculates the final predicted value of change in body weight with the following: the coefficient calculated based on the coefficient of determination, the temporary predicted value based on the newly acquired ketone body concentration, and the predicted value based on the predicted value of the ketone body concentration. Upon calculation of the final predicted value, processing proceeds to step S317.

In step S317, the calculation unit 18 creates an image indicating prediction of change in body weight displaying the predicted value of change in body weight calculated in step S303, S310, or S316 and causes the display unit 11 to display the image. Upon display of the image on the display unit 11, processing proceeds to step S318.

In step S318, the calculation unit 18 determines whether a ketone body concentration acquired in the past is stored in the storage unit 17. When such a ketone body concentration is stored, processing proceeds to step S319. Otherwise, the subroutine for prediction of change in body weight terminates.

In step S319, the calculation unit 18 determines whether the input unit 12 has detected input to display a graph of the predicted value of change in body weight. When such input is detected, processing proceeds to step S320. Otherwise, the subroutine for prediction of change in body weight terminates.

In step S320, the calculation unit 18 calculates the predicted value of change in body weight for periods of time from the current time to the designated time for which input was detected in step S301, creates a graph, and causes the display unit 11 to display the graph. The calculation of the predicted value of change in body weight may be made using any of the first formula for calculating a predicted value of change in body weight (Formula (2) in step S303), adjustment with an adjustment coefficient (Formulas (3) through (7) in step S310), or the second formula for calculating a predicted value of change in body weight (Formula (10) in step S316). Upon display of the graph, the subroutine for prediction of change in body weight terminates.

According to the apparatus for predicting change in a physical index as structured above in the present embodiment, the future predicted value of change in body weight can be calculated based on the ketone body concentration excreted by a subject. It is known that the ketone body concentration excreted by a living organism correlates with the energy of body fat burned from when body fat started to be burned in the living organism until the acquisition time of the ketone body concentration. The inventors discovered that the ketone body concentration excreted by a living organism also correlates with the future change in weight of body fat (or body weight). Based on the characteristics thus newly discovered by the inventors (correlation between ketone body concentration, length of time, and change in body weight), the apparatus 10 for predicting change in a physical index in the present embodiment calculates the predicted value of change in body weight, which conventionally was difficult, as described above.

Furthermore, the apparatus for predicting change in a physical index in the present embodiment calculates the future predicted value of change in body weight based on the product of the ketone body concentration excreted by the subject and a length of time from the current time to any designated time, thereby improving accuracy of the predicted value. As described above, the inventors discovered the correlation between the ketone body concentration excreted by a living organism and change in body weight. In particular, the inventors discovered that a first order or high-order polynomial having the product of ketone body concentration and length of time as a variable highly correlates to change in body weight. Based on the characteristics thus newly discovered by the inventors (correlation of the product of ketone body concentration and length of time with change in body weight), the apparatus for predicting change in a physical index in the present embodiment calculates the future predicted value of change in body weight to a high degree of accuracy, as described above.

The apparatus for predicting change in a physical index in the present embodiment calculates the predicted value of change in body weight also based on a ketone body concentration acquired in the past, thereby reflecting the characteristics of change in body weight of a particular subject and further improving accuracy of the predicted value. In particular, when a plurality of ketone body concentrations can be used, the tendency of change in the ketone body concentration of a particular subject can be reflected in the calculation of the predicted value of change in body weight by predicting the current ketone body concentration based on the actual past ketone body concentrations of the subject. Accordingly, the calculation accuracy of the predicted value for the subject can be greatly improved. When only one ketone body concentration can be used, then by simply reflecting the tendency of change in the ketone body concentration of the subject in the calculation of the predicted value of change in body weight, the accuracy of the predicted value can be improved as compared to when using only a predetermined formula for calculation.

The apparatus for predicting change in a physical index in the present embodiment determines the degree of increase in burning rate of body fat by the subject based on a newly acquired ketone body concentration and a past ketone body concentration, thereby allowing the subject to easily recognize the degree of the current status of body fat burning.

According to the apparatus for predicting change in a physical index in the present embodiment, the threshold used to determine the degree of increase in burning rate of body fat can be changed based on at least one of the subject's age and a past ketone body concentration. According to this structure, the threshold changes due to factors that cause the ease with which body fat is burned to vary, such as the subject's age, a past ketone body concentration, and the like. Therefore, the degree of increase in burning rate of body fat appropriate for the subject's age or past exercise status (past ketone body concentration) can be determined.

Although the present invention has been described by way of drawings and an embodiment, it is to be noted that various changes and modifications will be apparent to those skilled in the art based on the present disclosure. Accordingly, such changes and modifications are to be understood as included within the scope of the present invention.

For example, in the present embodiment, the designated time is established by the input unit 12 detecting input designating the time for which change in body weight is to be predicted, yet a different type of setting unit than the input unit may set the designated time for prediction. The designated time may, for example, be an actual time for which change in body weight is to be predicted, or be based on a program that sets a designated time in accordance with circumstances, such as a program that selects a time that is a certain period of time after the current time.

REFERENCE SIGNS LIST

10: Apparatus for predicting change in physical index
11: Display unit
12: Input unit
13: Acquisition unit
14: Bus
16: Timer
17: Storage unit
18: Calculation unit
A: Arrow
CG: Circular graph
IND: Indicator
MSG: Message

**Claims**

1. An apparatus (10) for predicting change in a physical index indicating body weight of a subject, comprising:

   an acquisition unit (13) configured to acquire a ketone body concentration excreted by the subject;
   a setting unit (12) configured to set a time at or later than the present;
   a storage unit (17) configured to store at least one ketone body concentration acquired by the acquisition unit (13); and
   a calculation unit (18) configured to (i) calculate, based on the ketone body concentration, on the set time and on a past ketone body concentration stored in the storage unit (17), a temporary predicted value of change in a physical index of the subject when the set time elapses, (ii) determine a degree of increase in burning rate of body fat by the subject based on a change between the past ketone body concentration and a current ketone body concentration newly acquired by the acquisition unit (13), (iii) select an adjustment coefficient corresponding to the determined degree of increase in burning rate of body fat, (iv) calculate a final predicted value of change in a physical index of the subject by correcting the temporary predicted value in accordance with the selected adjustment coefficient, and (v) control a display to output the calculated final predicted value of change in a physical index of the subject.

2. The apparatus (10) according to claim 1, wherein
   the calculation unit (18) is configured to adjust the predicted value of change in the physical index in accordance with a change between a past ketone body concentration stored in the storage unit (17) and a current ketone body concentration newly acquired by the acquisition unit (13).

3. The apparatus (10) according to claim 1, wherein
   the storage unit (17) is configured to store the ketone body concentration acquired by the acquisition unit (13) in combination with an acquisition time, and
   by taking a weighted average of i) the predicted value of change in the physical index calculated based on a plurality of past ketone body concentrations and acquisition times stored in the storage unit (17) and ii) the predicted value of change in the physical index calculated based on a current ketone body concentration newly acquired by the acquisition unit (13) and on the set time, the calculation unit (18) is configured to calculate the predicted value of change in the physical index based on the current ketone body concentration, the past ketone body concentrations, and the set time.

4. The apparatus (10) according to any one of claims 1 to 3, wherein the calculation unit (18) is configured to change, in accordance with age of the subject, a threshold of change in the ketone body concentration for determining the degree of increase in burning rate.

**5.** The apparatus (10) according to any one of claims 1 to 4, wherein the calculation unit (18) is configured to change, in accordance with the past ketone body concentration, a threshold of change in the ketone body concentration for determining the degree of increase in burning rate.

**6.** The apparatus (10) according to any one of claims 1 to 5, wherein the calculation unit (18) is configured to calculate the predicted value of change in the physical index based on a product of the ketone body concentration and a length of time from a current time to the set time.

**Patentansprüche**

**1.** Vorrichtung (10) zum Vorhersagen einer Änderung eines physischen Indexes, der das Körpergewicht einer Person angibt, umfassend:

eine Erfassungseinheit (13), die gestaltet ist, um eine Ketokörperkonzentration zu erfassen, die von der Person ausgeschieden wird;
eine Einstelleinheit (12), die gestaltet ist, um eine Zeit auf einen gegenwärtigen oder späteren Zeitpunkt einzustellen;
eine Speichereinheit (17), die gestaltet ist, um mindestens eine von der Erfassungseinheit (13) erfasste Ketokörperkonzentration zu speichern;
und
eine Recheneinheit (18), die gestaltet ist, um (i) auf der Grundlage der Ketokörperkonzentration zum eingestellten Zeitpunkt und einer früheren Ketokörperkonzentration, die in der Speichereinheit (17) gespeichert ist, einen vorläufigen Vorhersagewert der Änderung eines physischen Indexes der Person nach Ablauf der eingestellten Zeit zu berechnen, (ii) einen Grad des Anstiegs der Körperfettverbrennungsrate der Person auf der Grundlage einer Veränderung zwischen der früheren Ketokörperkonzentration und einer von der Erfassungseinheit (13) neu erfassten gegenwärtigen Ketokörperkonzentration zu bestimmen, (iii) einen Anpassungskoeffizienten auszuwählen, der dem bestimmten Grad des Anstiegs der Körperfettverbrennungsrate entspricht, (iv) einen endgültigen Vorhersagewert der Änderung eines physischen Indexes der Person durch Korrektur des vorläufigen Vorhersagewerts in Übereinstimmung mit dem ausgewählten Anpassungskoeffizienten zu berechnen, und (v) eine Anzeige zu steuern, um den berechneten endgültigen Vorhersagewert der Änderung eines physischen Indexes der Person auszugeben.

**2.** Vorrichtung (10) nach Anspruch 1, wobei die Recheneinheit (18) gestaltet ist, um den Vorhersagewert der Änderung des physischen Indexes in Übereinstimmung mit einer Veränderung zwischen einer früheren Ketokörperkonzentration, die in der Speichereinheit (17) gespeichert ist, und einer von der Erfassungseinheit (13) neu erfassten gegenwärtigen Ketokörperkonzentration anzupassen.

**3.** Vorrichtung (10) nach Anspruch 1, wobei die Speichereinheit (17) gestaltet ist, um die von der Erfassungseinheit (13) erfasste Ketokörperkonzentration zusammen mit einem Erfassungszeitpunkt zu speichern, und wobei die Recheneinheit (18) gestaltet ist, um durch Bilden eines gewichteten Mittelwerts i) des Vorhersagewerts der Änderung des physischen Indexes, der auf der Grundlage einer Vielzahl von früheren Ketokörperkonzentrationen und Erfassungszeitpunkten, die in der Speichereinheit (17) gespeichert sind, berechnet wird, und ii) des Vorhersagewerts der Änderung des physischen Indexes, der auf der Grundlage einer von der Erfassungseinheit (13) neu erfassten gegenwärtigen Ketokörperkonzentration und der eingestellten Zeit berechnet wird, den Vorhersagewert der Änderung des physischen Indexes auf der Grundlage der gegenwärtigen Ketokörperkonzentration, der früheren Ketokörperkonzentrationen und der eingestellten Zeit zu berechnen.

**4.** Vorrichtung (10) nach einem der Ansprüche 1 bis 3, wobei die Recheneinheit (18) gestaltet ist, um in Übereinstimmung mit dem Alter der Person einen Schwellenwert der Änderung der Ketokörperkonzentration zum Bestimmen des Grads des Anstiegs der Verbrennungsrate zu ändern.

**5.** Vorrichtung (10) nach einem der Ansprüche 1 bis 4, wobei die Recheneinheit (18) gestaltet ist, um in Übereinstimmung mit der früheren Ketokörperkonzentration einen Schwellenwert der Änderung der Ketokörperkonzentration zum Bestimmen des Grads des Anstiegs der Verbrennungsrate zu ändern.

**6.** Vorrichtung (10) nach einem der Ansprüche 1 bis 5, wobei die Recheneinheit (18) gestaltet ist, um den Vorhersagewert der Änderung des physischen Indexes auf der Grundlage eines Produkts aus der Ketokörperkonzentration und einer Zeitspanne von einem gegenwärtigen Zeitpunkt bis zum eingestellten Zeitpunkt zu berechnen.

**Revendications**

1.  Appareil (10) pour prédire le changement d'un indice physique indiquant le poids corporel d'un sujet, comprenant :

    une unité d'acquisition (13) configurée pour acquérir une concentration des corps cétoniques excrétés par le sujet ;
    une unité de réglage (12) configurée pour régler un moment présent ou ultérieur ;
    une unité de stockage (17) configurée pour stocker au moins une concentration des corps cétoniques acquise par l'unité d'acquisition (13) ;
    et
    une unité de calcul (18) configurée pour (i) calculer, sur la base de la concentration des corps cétoniques, au moment spécifié et selon une concentration des corps cétoniques antérieure stockée dans l'unité de stockage (17), une valeur prédite temporaire du changement d'un indice physique du sujet lorsque le temps spécifié s'est écoulé, (ii) déterminer un degré d'augmentation du taux de combustion de la graisse corporelle par le sujet selon le changement entre la concentration des corps cétoniques antérieure et une concentration des corps cétoniques actuelle nouvellement acquise par l'unité d'acquisition (13), (iii) sélectionner un coefficient d'ajustement correspondant au degré d'augmentation déterminé du taux de combustion de la graisse corporelle, (iv) calculer une valeur prédite finale du changement d'un indice physique du sujet en rectifiant la valeur prédite temporaire conformément au coefficient d'ajustement sélectionné, et (v) contrôler un dispositif d'affichage pour indiquer la valeur prédite finale calculée du changement d'un indice physique du sujet.

2.  Appareil (10) selon la revendication 1, dans lequel l'unité de calcul (18) est configurée pour ajuster la valeur prédite du changement d'un indice physique conformément à un changement entre une concentration des corps cétoniques antérieure stockée dans l'unité de stockage (17) et une concentration des corps cétoniques actuelle nouvellement acquise par l'unité d'acquisition (13) .

3.  Appareil (10) selon la revendication 1, dans lequel l'unité de stockage (17) est configurée pour stocker la concentration des corps cétoniques acquise par l'unité d'acquisition (13) en combinaison avec un temps d'acquisition, et en prenant une moyenne pondérée de i) la valeur prédite du changement de l'indice physique calculée sur la base d'une pluralité de concentrations de corps cétoniques antérieures et de temps d'acquisition stockés dans l'unité de stockage (17) et ii) la valeur prédite du changement de l'indice physique calculée sur la base d'une concentration des corps cétoniques actuelle nouvellement acquise par l'unité d'acquisition (13) et du moment spécifié, l'unité de calcul (18) est configurée pour calculer la valeur prédite du changement de l'indice physique sur la base de la concentration des corps cétoniques actuelle, les concentrations des corps cétoniques antérieures, et le moment spécifié.

4.  Appareil (10) selon l'une quelconque des revendications 1 à 3, dans lequel l'unité de calcul (18) est configurée pour changer, en fonction de l'âge du sujet, un seuil de changement de la concentration des corps cétoniques pour déterminer le degré d'augmentation du taux de combustion.

5.  Appareil (10) selon l'une quelconque des revendications 1 à 4, dans lequel l'unité de calcul (18) est configurée pour changer, en fonction de la concentration des corps cétoniques antérieure, un seuil de changement de la concentration des corps cétoniques pour déterminer le degré d'augmentation du taux de combustion.

6.  Appareil (10) selon l'une quelconque des revendications 1 à 5, dans lequel l'unité de calcul (18) est configurée pour calculer la valeur prédite de changement de l'indice physique sur la base d'un produit de la concentration des corps cétoniques et d'une durée allant d'un moment actuel à un moment spécifié.

# FIG. 1

# FIG. 2

# FIG. 3

11

12 — Current burning rate of
body fat: about 3.0 g per hour

12 —

Current direction of change
in body weight: decreasing

12

10

# FIG. 4

11

Current degree of body fat burning

12

12

12

IND

10

# FIG. 5

11     MSG

12

Super
speed up

A

Non-consecutive days.
Result only for
reference.

12

CG

10

CG

Overexertion
warning!

Down

Super
speed up

Stable

Speed up

# FIG. 6

11

12

12

If you keep up this pace,
in three days you'll lose 1 kg?!

12

10

# FIG. 7

11

12

If you keep up this pace, in three days your fat percentage will go down 3%?

12

12

10

# FIG. 8

11

12

12

If you keep up this pace, you'll reach your goal in two days?!

12

10

## FIG. 9

```
                    ( START )
                        │
                        ▼
S100 ──┤ Acquire ketone concentration │
                        │
                        ▼
S101 ──< Input to determine state? >── NO ──┐
                        │ YES                 │
                        ▼                     │
S102 ──┤ Determine state │                   │
                        │◄────────────────────┘
                        ▼
S103 ──< Past ketone body           >── NO ──┐
         concentration stored?                │
                        │ YES                 │
                        ▼                     │
S104 ──< Input to determine degree  >── NO ───┼──►
              of increase?                    │
                        │ YES                 │
                        ▼                     │
S200 ──┤ Determine degree of increase │       │
         in fat burning rate                  │
                        │◄────────────────────┘
                        ▼
S105 ──< Input to predict change    >── NO ──┐
              in body weight?                 │
                        │ YES                 │
                        ▼                     │
S300 ──┤ Predict change in │                  │
              body weight                     │
                        │◄────────────────────┘
                        ▼
                    ( END )
```

# FIG. 10

```
        ╭─────────────────────────────────────╮
        │ Subroutine for determining the degree │
        │ of increase in fat burning rate (S200) │
        ╰─────────────────────────────────────╯
                        │
                        ▼
        ┌─────────────────────────────────────┐
S201 ~  │       Read most recent ketone        │
        │        body concentration            │
        └─────────────────────────────────────┘
                        │
                        ▼
        ┌─────────────────────────────────────┐
S202 ~  │          Read decision table         │
        └─────────────────────────────────────┘
                        │
                        ▼
        ┌─────────────────────────────────────┐
S203 ~  │        Select degree of increase     │
        └─────────────────────────────────────┘
                        │
                        ▼
        ┌─────────────────────────────────────┐
S204 ~  │         Calculate length of time     │
        └─────────────────────────────────────┘
                        │
                        ▼
S205 ~  ⟨ Length of time > time threshold? ⟩──── NO ──┐
                        │ YES                          │
                        ▼                              │
        ┌─────────────────────────────────────┐        │
S206 ~  │         Determine whether            │        │
        │         to attach message            │        │
        └─────────────────────────────────────┘        │
                        │◄─────────────────────────────┘
                        ▼
        ┌─────────────────────────────────────┐
S207 ~  │           Display image              │
        └─────────────────────────────────────┘
                        │
                        ▼
                   ╭──────────╮
                   │  RETURN  │
                   ╰──────────╯
```

*FIG. 11*

```
        ┌────────────────────────────┐
        │ Subroutine for predicting change │
        │   in body weight (S300)    │
        └────────────────────────────┘
                      │
          ┌───────────▼──────────────┐  NO
    ┌─────│ Input of designated time detected? │───S301
    │     └──────────────────────────┘
    │                 │ YES
    │     ┌───────────▼──────────────┐
    │     │  Calculate length of time │───S302
    │     └──────────────────────────┘
    │                 │
    │     ┌───────────▼──────────────┐
    │     │    Calculate predicted value │───S303
    │     │  of change in body weight │
    │     └──────────────────────────┘
```

S304 — Past ketone body concentration stored?  — NO

YES

S305 — Only one ketone body concentration stored?  — One

More than one

| S311 — Create temporary formula for calculation | S306 — Read decision table |
| S312 — Exclude unsuitable ketone body concentrations | S307 — Select degree of increase |
| S313 — Create formula for final calculation | S308 — Read decision table |
| S314 — Predict ketone body concentration immediately before time of prediction | S309 — Select adjustment coefficient |
| S315 — Calculate coefficient of determination | S310 — Calculate final predicted value by adjustment |
| S316 — Calculate the final predicted value using ketone body concentration | |

S317 — Display image

S318 — Past ketone body concentration stored?  — NO

YES

S319 — Input to display graph detected?  — NO

YES

S320 — Display graph

RETURN

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001349888 A
- WO 03079890 A1
- WO 2007062663 A1
- WO 2013046810 A1
- US 6506152 B1
- US 2007083335 A1
- WO 2004088304 A2